# EUROPEAN PATENT SPECIFICATION

(11) **EP 2 723 414 B1**
(45) Date of publication and mention of the grant of the patent: **12.04.2017**
(21) Application number: 12802456.9
(22) Date of filing: 19.06.2012
(51) Int. Cl.: A61M 1/00, A61B 17/02, A61F 2/12, A61B 17/34, A61B 17/00

(54) **IMPLANT INSERTION DEVICE**
VORRICHTUNG ZUM EINSETZEN VON IMPLANTATEN
DISPOSITIF D'INSERTION D'IMPLANT

(30) Priority: 21.06.2011 US 201161499490 P
(43) Date of publication of application: 30.04.2014
(73) Proprietor: University Hospitals Cleveland Medical Center, Cleveland, OH 44106 (US)
(72) Inventor: ZOCHOWSKI, Christopher, G., Cleveland, OH 44118 (US)
(74) Representative: McNab, Donald C.
(86) International application number: PCT/US2012/043060
(87) International publication number: WO 2012/177587

(56) References cited:
- WO-A2-01/50943
- US-A- 5 201 779
- US-A- 5 723 006
- US-A- 5 723 006
- US-A1- 2006 184 100
- US-A1- 2007 276 484
- US-A1- 2009 177 165
- US-A1- 2009 204 107
- US-A1- 2010 249 516
- US-A1- 2011 035 003
- US-A1- 2011 035 003
- US-A1- 2011 082 546

## Description

### FIELD OF THE INVENTION

The present invention generally relates to an implant insertion device, and particularly, to a breast implant insertion device and method of using thereof.

### BACKGROUND OF THE INVENTION

Breast implants may be positioned within the chest, for example, in one of three positions: (1) implant over the pectoralis major muscle and under the breast tissue (subglandular); (2) implant partially under the muscle (partial submuscular or "dual plane"); and (3) implant completely under the muscle (submuscular). The subglandular placement puts the implant directly behind the breast tissue and mammary gland and in front of the pectoralis major muscle. This placement requires the least amount of dissection and yields the quickest recovery in comparative studies.

Partial submuscular placement involves placing the implant under the pectoralis major muscle. Because of the structure of this muscle, the implant is only partially covered. Completely submuscular placement puts the implant firmly behind the muscle. The implant is placed behind the pectoralis major muscle and behind all of the supporting fascia (connective tissue) and non-pectoral muscle groups.

Regardless of location of the implant, in the case of breast augmentation the surgery is carried out through an incision placed to minimize visibility of the resultant scar. The incision is made in, but is not limited to, one of three areas: (1) peri-areolar incision; (2) inframammary fold incision; and (3) transaxillary incision. The peri-areolar incision enables the surgeon to place the implant in the subglandular, partial submuscular or completely submuscular position, with the implant being inserted, or removed, through the incision. Like the peri-areolar incision, the inframammary fold incision provides for all three placement types and both insertion and removal of the implant through the incision. The incision is made in the crease under the breast, allowing for discreet scarring. Once the incision is made, the implant is inserted and worked vertically into place after creation of an appropriate sized pocket.

The transaxillary incision is made in the armpit. The incision is made in the fold of the armpit and a channel is dissected to gain access to the desired plane. The implant is inserted into the channel and worked into place. Like the peri-areolar and crease incisions, the armpit incision can be used for implant placement in all the previously described planes. Once the incision is created, the surgeon dissects a path through the tissue to the final destination of the implant. Once that path has been created, the tissue and/or muscle (depending on placement) is separated to create a pocket for the implant.

Since breast implants are usually placed into the body through incisions considerably smaller than the implant, it is a challenge to introduce them. With friction at the interface between the surface of the implants and the wound margins (body tissue), it is difficult to introduce the implants. Increased manipulation of both implants and patient tissue often results in trauma to both implants and patient tissue, thereby increasing the risk associated with the procedure both in terms of immediate consequences as well as delayed structural failure and the implications deriving therefrom.

Postoperative infection has also been a consequence of the need to manipulate the implant into place. If this occurs, the removal of the implant may be warranted and permanent disfigurement may result. in addition, bacterial seeding of the wound is postulated to lead to complications such as capsular contracture. The implant may, for example, become seeded with bacteria if it contacts the skin of the patient. Measures are taken in the operating room to avoid such risks. For example, antibiotic solution(s) may be used in the wound, the surgeon's gloves may be changed, and efforts may be made to reduce contact of the implant with the skin. However, such measures suffer from a variety of deficiencies and still provide opportunities for infection or bacterial seeding to occur. US 5,723,006 describes a tool for introducing an implant through an incision in a body utilizing a tube having a hole through which the implant is pressed.

Accordingly, it would be desirable to provide an implant insertion device capable of overcoming these and other complications alone or in combination.

Additional features and advantages of the invention will be set forth in the description that follows, and in part will be apparent from the description, or may be learned by practice of the invention. The objectives and other advantages of the invention will be realized and attained by the structure particularly pointed out in the written description and claims hereof as well as the appended drawings. It is to be understood that both the foregoing general description and the following detailed description are exemplary and explanatory and are intended to provide further explanation of the invention as claimed.

### SUMMARY OF THE INVENTION

The invention is defined by the appended claims.

In an embodiment, an implant insertion device is provided comprising a body defining an interior cavity capable of receiving an implant therein, a neck having an end extending from the body and insertable in an incision of a patient, and an aperture on the neck and in communication with the cavity, where the aperture is positionable substantially coaxially with the incision and the implant is transferable from the cavity to exit out the aperture into the patient. The neck, body, and combination thereof may be foldable or stretchable. The end may be insertable through the incision without contacting the skin of the patient. The body may be flexible, substantially transparent, and combinations thereof. The body may further comprise a second aperture in communication with the cavity for insertion of the implant therein. The second aperture may be larger than the implant. The second aperture may be selectively closeable. The device may further comprise a port in the body that is extendable through at least a portion of the cavity and the first aperture. The port may be glove shaped. The device may further comprise a removable cover over the first aperture. The device may further comprise an implant positioned in the cavity. The implant may be a breast implant. The device may further comprise a compartment capable of being placed in fluid communication with the body. The compartment may contain a fluid including, but limited to a lubricant, disinfectant, sterilizer, antibiotic, antimicrobial, and combinations thereof. The compartment may be positioned in the cavity. The compartment may be selectively openable to release the fluid in the cavity.

In an embodiment, an implant insertion device comprises a body defining an interior cavity capable of receiving an implant therein, an aperture in communication with the cavity and positionable substantially coaxially with an incision in a patient, where the implant is transferable from the cavity to exit out the aperture into the patient, and a member extending from the body for selectively engaging the patient. The member may engage the patient to maintain alignment of the aperture with the incision, to prevent withdrawal of the device from the patient during insertion of the implant, to prevent over insertion of the device in the patient, and combinations thereof. The member may engage the external surface of the patient's skin. The member may engage a surface inside the patient. The member may be a flexible ring. The member may be a tab. The member may engage the internal surface opposite the external surface of the skin. The device may further comprise a second member extending from the body for engaging the external surface of the patient's skin. The second member may engage the external surface of the patient's skin to maintain alignment of the aperture with the incision, to prevent withdrawal of the device from the patient during insertion of the implant, to prevent over insertion of the device in the patient, and combinations thereof. The second member may be selectively positionable from a first non-engagement position to a second engagement position. The first member and the second member may compress or sandwich the skin therebetween when the second member is positioned in the second engagement position. The second member may be a flexible ring. The second member may have a diameter greater than the diameter of the first member, substantially equal to the diameter of the first member, or smaller than the diameter of the first member.

In an embodiment, an implant insertion device comprises a body defining an interior cavity capable of receiving an implant therein, an aperture in communication with the cavity and positionable substantially coaxially with an incision in a patient, where the implant is transferable from the cavity to exit out the aperture into the patient, and a neck extending from the body for selectively engaging the incision to maintain the incision in an open position. The aperture may be positioned on the neck. The neck may be moveable from a first non-engagment position to a second engagement position. The neck is insertable in said incision without substantially contacting the skin while in said first non-engagment position. The neck may comprise a first leg for engaging a first side of the incision, and a second leg for engaging a second side of the incision. The first leg and the second leg may be moveable from a first non-engagement position to a second engagement position. The device may further comprise a biasing member for selectively maintaining the first leg and the second leg in the second engagement position. The neck may maintain alignment of the aperture with the incision. The diameter of the neck in the first position is smaller than the diameter of the neck in the second position.

In one embodiment the invention relates to an implant insertion device comprising: a) a body defining an interior cavity capable of receiving an implant therein, b) an aperture in communication with the cavity, c) positionable substantially coaxially with an incision in a patient, where said implant is transferable from the cavity to exit out the aperture into the patient, and d) a member extending from the body for selectively engaging the patient. In one embodiment the invention relates to the method of using the device of described above to deliver an implant to a patient. In one embodiment the member may engage the patient to maintain alignment of the aperture with the incision, to prevent withdrawal of the device from the patient during insertion of the implant, to prevent over insertion of the device in the patient, and combinations thereof. In one embodiment the member may engage the external surface of the patient's skin. In one embodiment the member may engage a surface inside the patient. In one embodiment the member may be a flexible ring. In one embodiment the member may be a tab. In one embodiment member may engage the internal surface opposite the external surface of the skin. In one embodiment the device may further comprise a second member extending from the body for engaging the external surface of the patient's skin. In one embodiment the second member may engage the external surface of the patient's skin to maintain alignment of the aperture with the incision, to prevent withdrawal of the device from the patient during insertion of the implant, to prevent over insertion of the device in the patient, and combinations thereof. In one embodiment the invention relates to method of using the device described above to deliver an implant to a patient. In one embodiment the second member may be selectively positionable from a first non-engagement position to a second engagement position. In one embodiment the first member and the second member may compress or sandwich the skin therebetween when the second member is positioned in the second engagement position. In one embodiment the second member may be a flexible ring. In one embodiment the second member may have a diameter greater than the diameter of the first member, substantially equal to the diameter of the first member, or smaller than the diameter of the first member. In one embodiment the invention relates to method of using the device described above to deliver an implant to a patient.

In one embodiment the invention relates to an implant insertion device comprising: a) a body defining an interior cavity capable of receiving an implant therein, b) a neck having an end extending from the body and insertable in an incision of a patient, c) an aperture on the neck and in communication with the cavity, where said aperture is positionable substantially coaxially with the incision, and d) said implant is transferable from the cavity to exit out the aperture into the patient. In one embodiment the invention relates to method of using the device described above to deliver an implant to a patient. In one embodiment the neck, body, and combination thereof may be foldable or stretchable. In one embodiment the end may be insertable through the incision without contacting the skin of the patient. In one embodiment the body may be flexible, substantially transparent, and combinations thereof. In one embodiment the body may further comprise a second aperture in communication with the cavity for insertion of the implant therein. In one embodiment the second aperture may be larger than the implant. In one embodiment the second aperture may be selectively closeable. In one embodiment the device may further comprise a port in the body that is extendable through at least a portion of the cavity and the first aperture. In one embodiment the port may be glove shaped. In one embodiment the device may further comprise a removable cover over the first aperture. In one embodiment the device may further comprise an implant positioned in the cavity. In one embodiment the implant may be a breast implant. In one embodiment the device may further comprise a compartment capable of being placed in fluid communication with the body. In one embodiment the compartment may contain a fluid including, but limited to a lubricant, disinfectant, sterilizer, antibiotic, antimicrobial, and combinations thereof. In one embodiment the compartment may be positioned in the cavity. In one embodiment the compartment may be selectively openable to release the fluid in the cavity. In one embodiment the invention relates to method of using the device described above to deliver an implant to a patient.

In one embodiment the invention relates to an implant insertion device comprising: a) a body defining an interior cavity capable of receiving an implant therein, b) an aperture in communication with the cavity and positionable substantially coaxially with an incision in a patient, where the implant is transferable from the cavity to exit out the aperture into the patient, and c) a neck extending from the body for selectively engaging the incision to maintain the incision in an open position. One example relates to method of using the device described above to deliver an implant to a patient. In one embodiment said aperture may be positioned on the neck. In one embodiment said neck may be moveable from a first non-engagment position to a second engagement position. In one embodiment said neck is insertable in said incision without substantially contacting the skin while in said first non-engagment position. In one embodiment said neck may comprise a first leg for engaging a first side of the incision, and a second leg for engaging a second side of the incision. In one embodiment said first leg and said second leg may be moveable from a first non-engagement position to a second engagement position. In one embodiment said device further comprises a biasing member for selectively maintaining the first leg and the second leg in the second engagement position. In one embodiment said neck may maintain alignment of the aperture with the incision. In one embodiment the diameter of the neck in the first position is smaller than the diameter of the neck in the second position. In one embodiment the invention relates to method of using the device described above to deliver an implant to a patient.

### BRIEF DESCRIPTION OF THE SEVERAL VIEWS OF THE DRAWINGS

Objects and advantages, together with the operation of the invention, may be better understood by reference to the following detailed description taken in connection with the following illustrations, wherein:
Figure 1 is a side view of an implant insertion device and a cross-sectional view of an incision in the tissue of a patient.
Figure 2A, Figure 2B, Figure 2C, and Figure 2D are side views of the body of the implant insertion device.
Figure 3A and Figure 3B are side views of the implant insertion device with a removable cover.
Figure 4 is a side view of the implant insertion device with a fluid in the cavity.
Figure 5 is a side view of the implant insertion device provided with a compartment containing the fluid.
Figure 6 is a side view of the implant insertion device with a second aperture.
Figure 7A is a side view of the implant insertion device with a selectively closeable second aperture in an open position.
Figure 8B is a side view of the implant insertion device with a selectively closeable second aperture in a closed position.

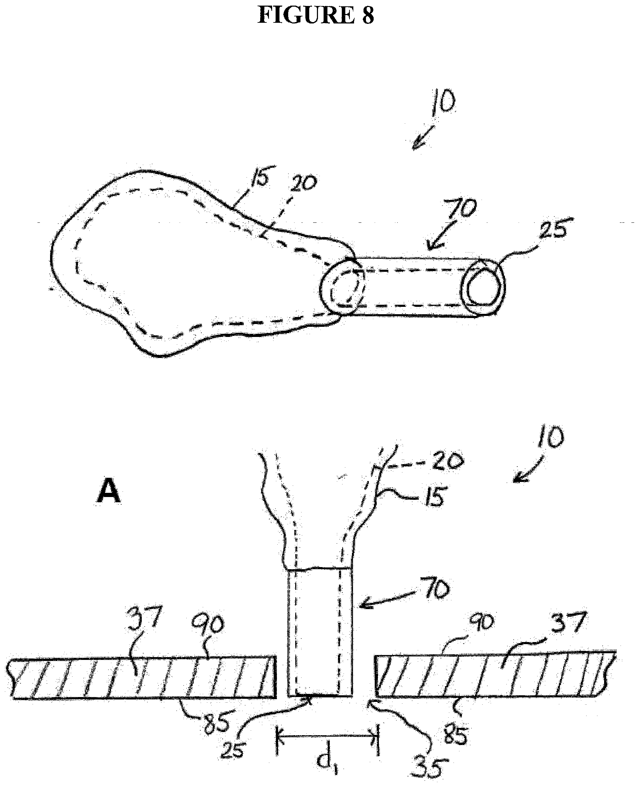

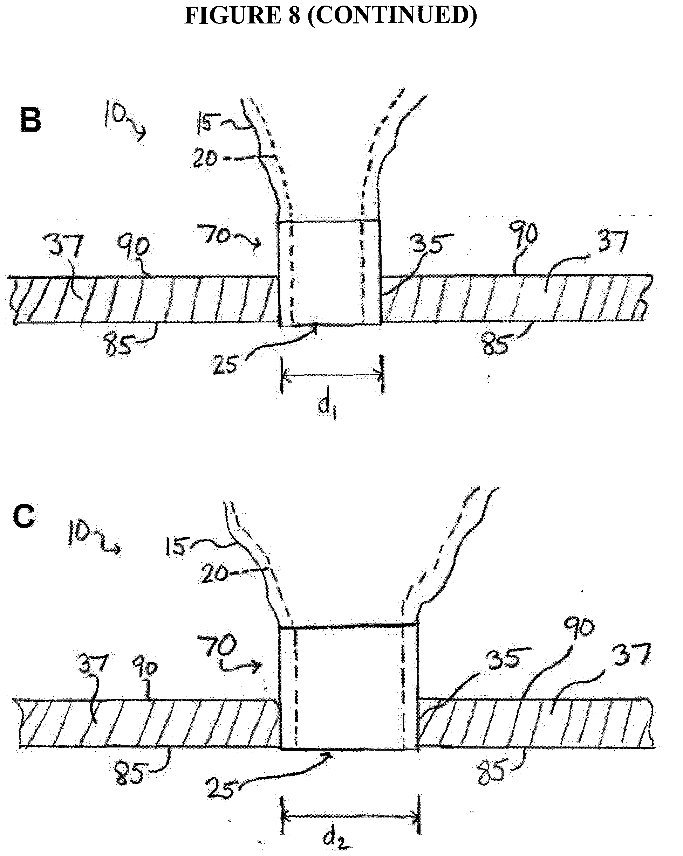

Figure 8 is a side view of the implant insertion device with a neck.
Figure 8A is a side view of the implant insertion device with a neck in a first non-engagement position.
Figure 8B is a side view of the implant insertion device of Figure 8A with the neck in a second engagement position to maintain the incision in an open position.
Figure 8C is a side view of the implant insertion device of Figure 8A with the neck in the engagement position to expand the incision.

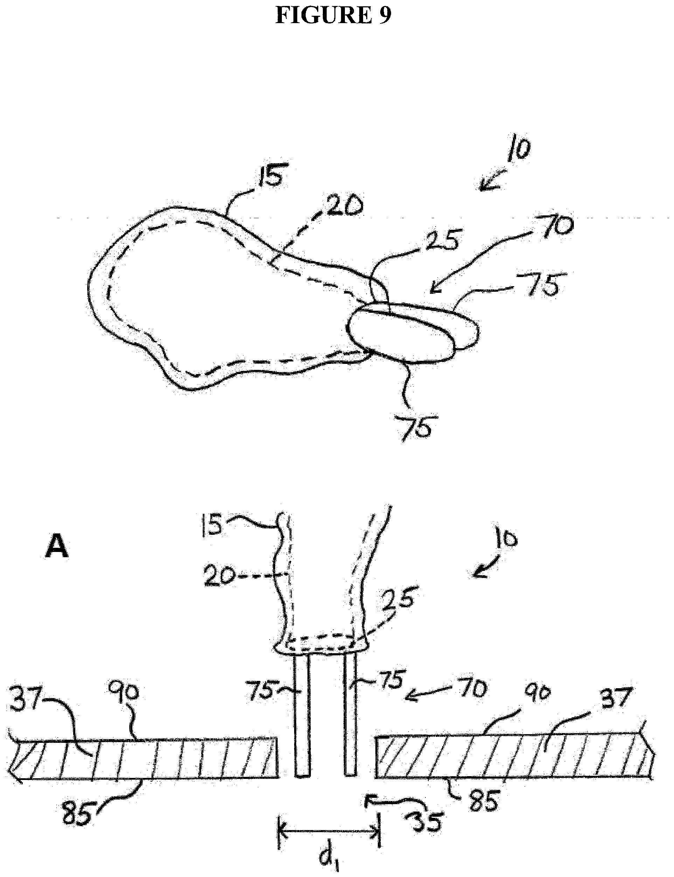

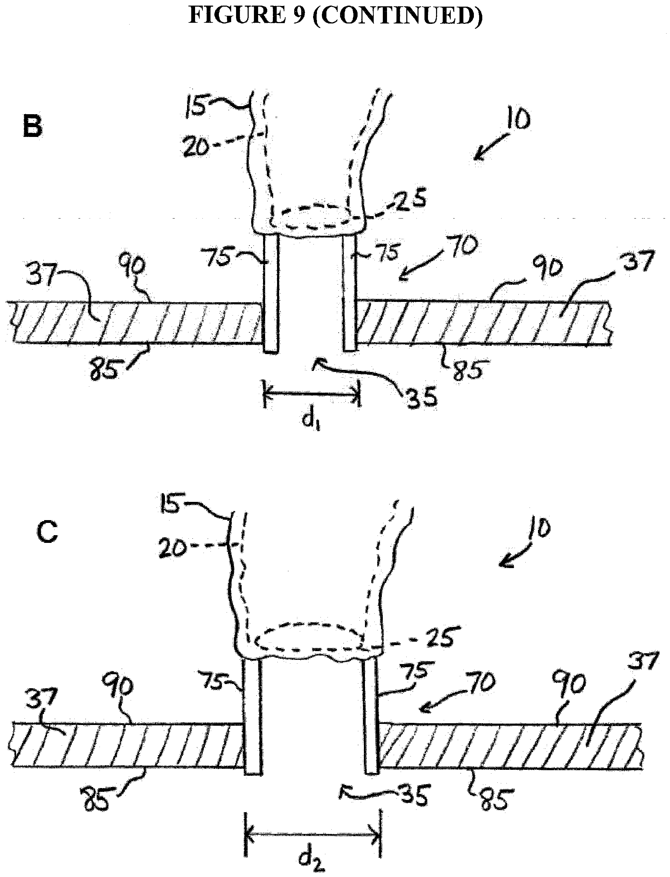

Figure 9 is a side view of the implant insertion device with a neck comprising legs.
Figure 9A is a side view of the implant insertion device provided with the neck in a first non-engagement position.
Figure 9B is a side view of the implant insertion device of Figure 9A with the neck in a second engagement position.
Figure 9C is a side view of the implant insertion device of Figure 9A with the neck in the engagement position.

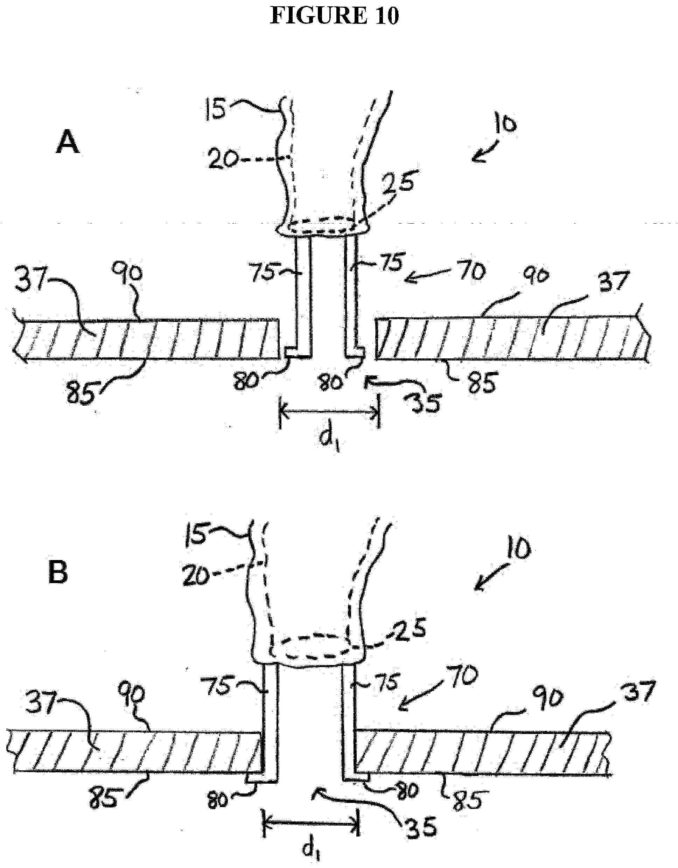

Figure 10A is a side view of the implant insertion device provided in Figure 9A with a first member extending from the neck.
Figure 10B is a side view of the implant insertion device provided in Figure 9B with the first member extending from the neck.

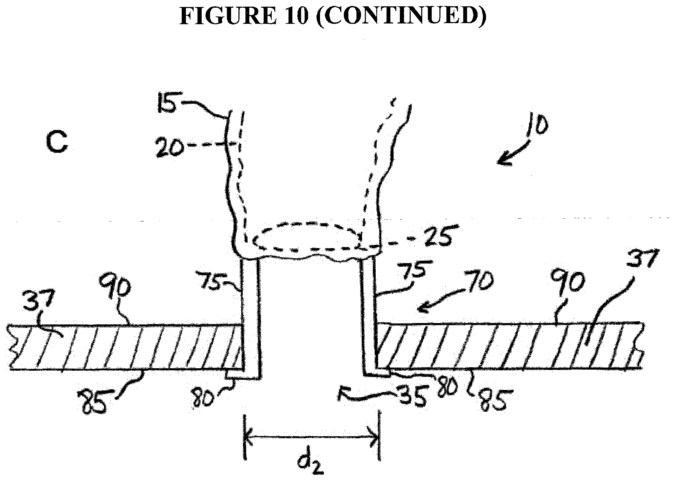

Figure 10C is a side view of the implant insertion device of Figure 9C with the first member extending from the neck.

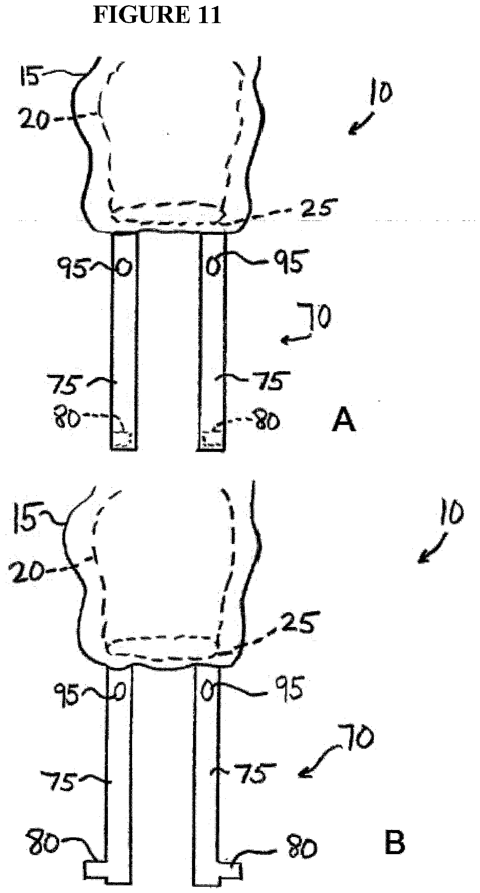

Figure 11A is a side view of the implant insertion device provided with the first member in a non-engagment position.
Figure 11B is a side view of the implant insertion device provided with the first member in an engagement position.

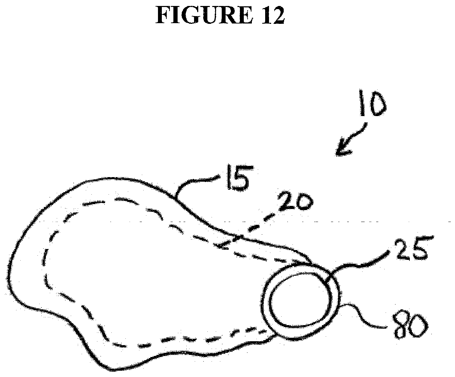

Figure 12 is a side view of the implant insertion device with the first member secured to the body.

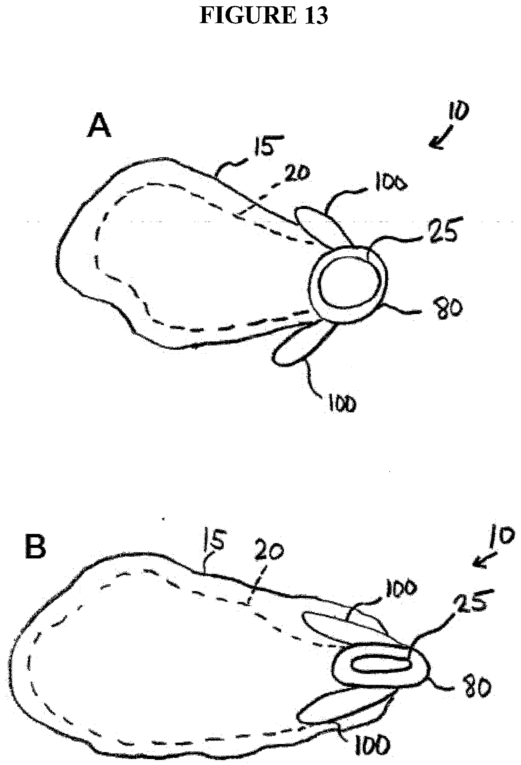

Figure 13A is a side view of the implant insertion device of Figure 12 with a leg extending from the first member.

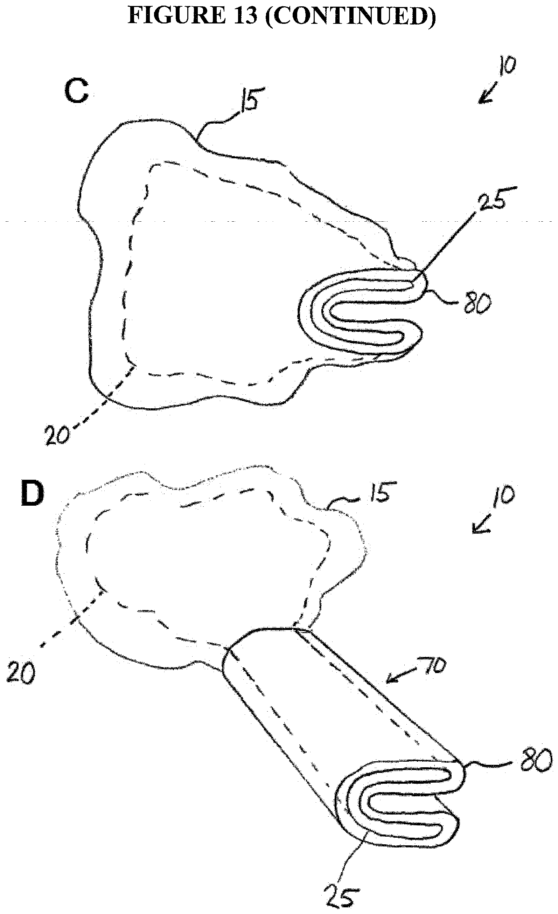

Figure 13B is a side view of the implant insertion device of Figure 13 with the first member in a non-engagement position.
Figure 13C is a side view of the implant insertion device of Figure 13 with a portion of the body and the first member in a non-engagement position.
Figure 13D is a side view of the implant insertion device with the neck and the first member in a non-engagement position.

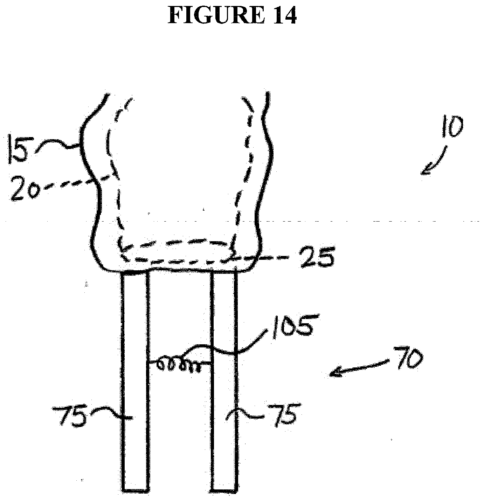

Figure 14 is a side view of the implant insertion device provided with a biasing member.

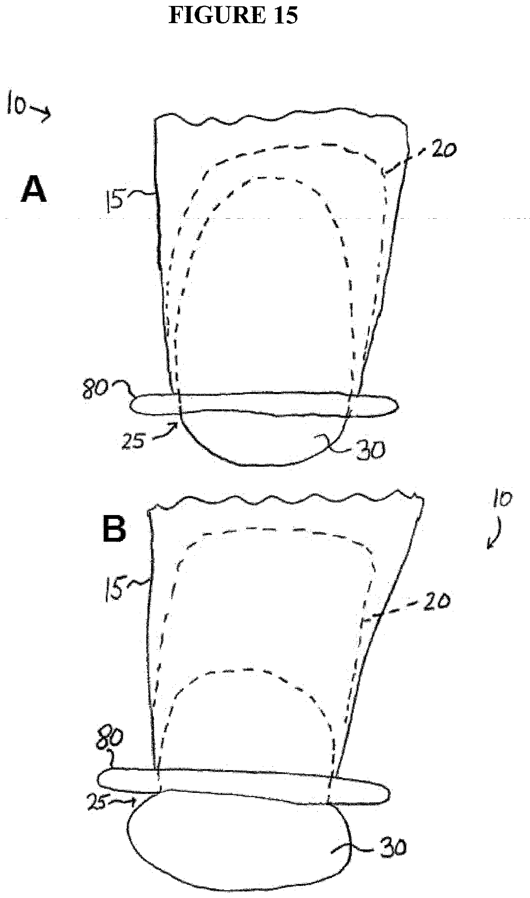

Figure 15A is a side view of the implant insertion device with the implant positioned partially therein.
Figure 15B is a side view of the implant insertion device of Figure 15A with the implant extruding outward from the cavity.

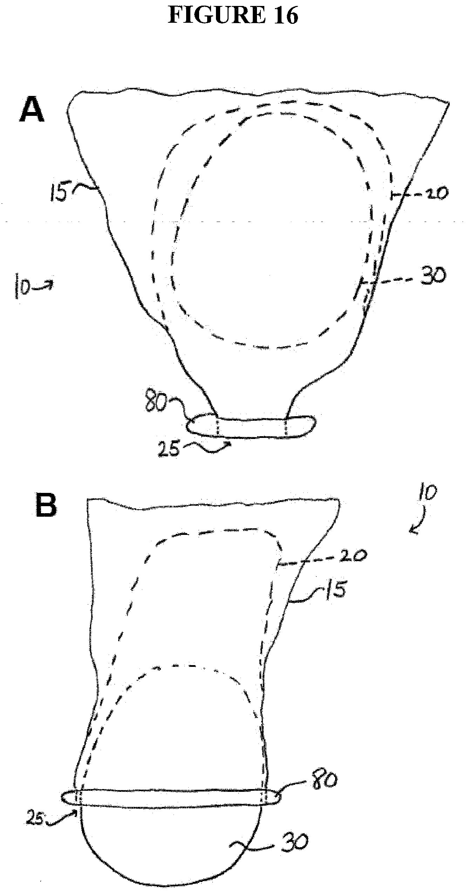

Figure 16A is a side view of the implant insertion device with the implant positioned in the cavity.
Figure 16B is a side view of the implant insertion device of Figure 16A with the implant exiting therefrom.

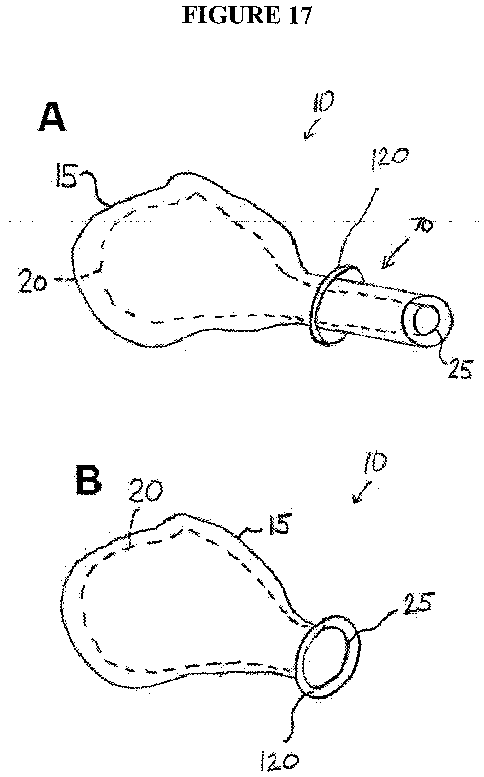

Figure 17A and Figure 17B are side views of the implant insertion device provided with a second member.

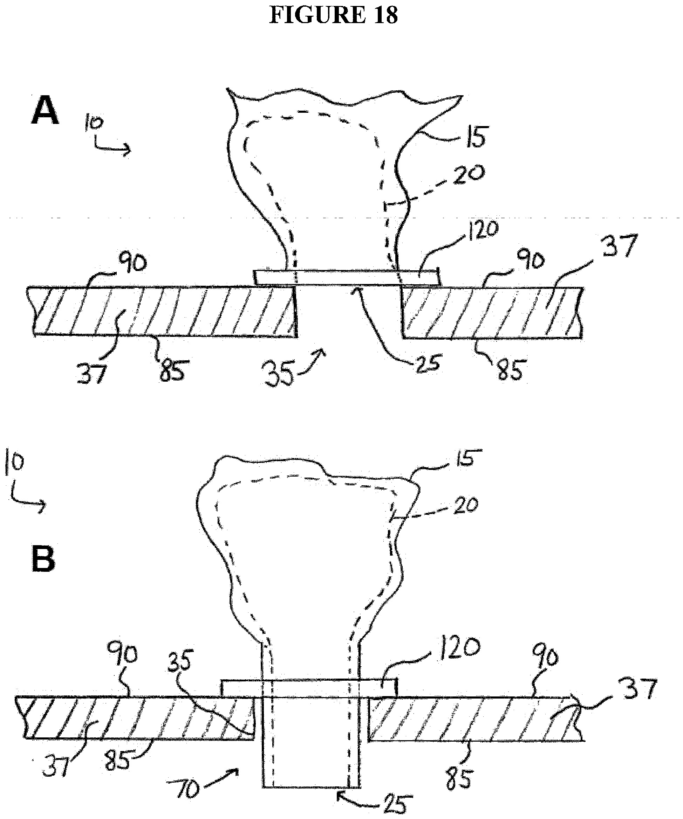

Figure 18A and Figure 18B are side views of the implant insertion devices of Figure 17A and 17B with the second member engaging the external surface of the patient's skin.

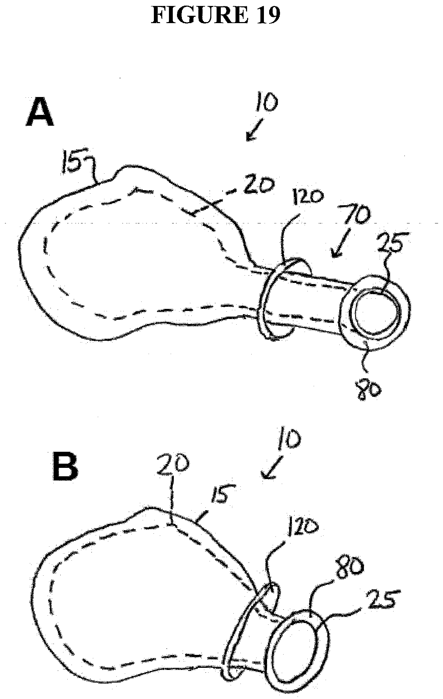

Figure 19A and Figure 19B are side views of the implant insertion device provided with the first member and the second member.

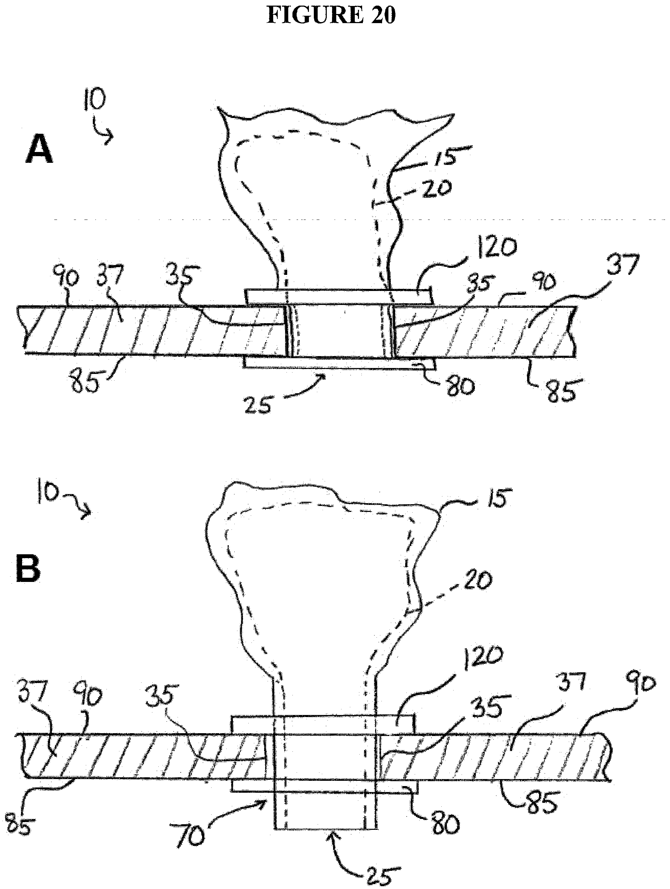

Figure 20A and Figure 20B are side views of the implant insertion devices of Figure 19A and Figure 19B with the first and second members engaging the tissue of the patient.

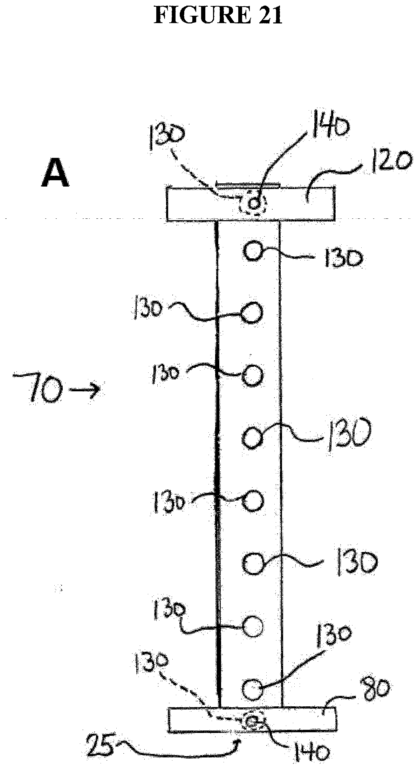

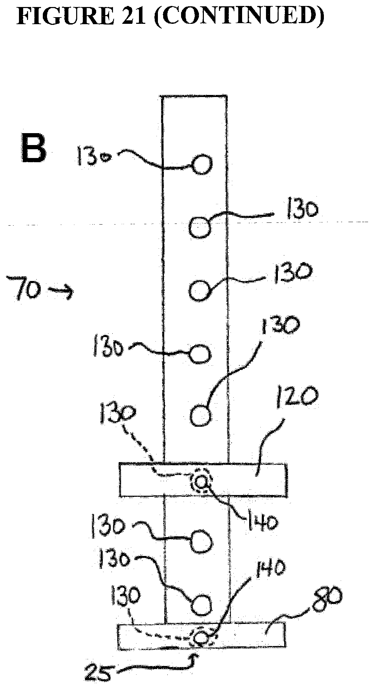

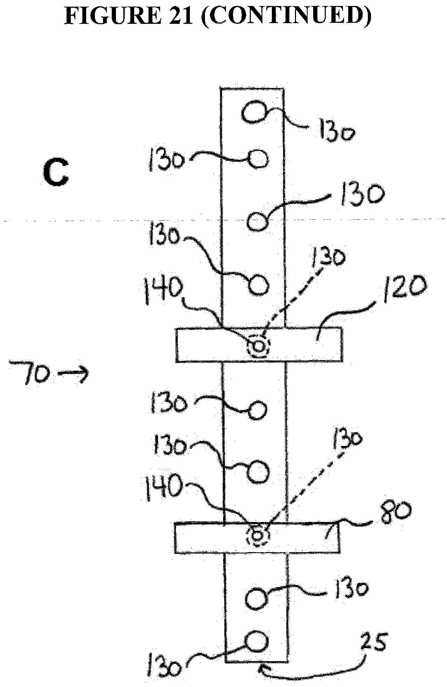

Figure 21A, Figure 21B, and Figure 21C are side views of the neck of the implant insertion device with selectively positionable first and second members.

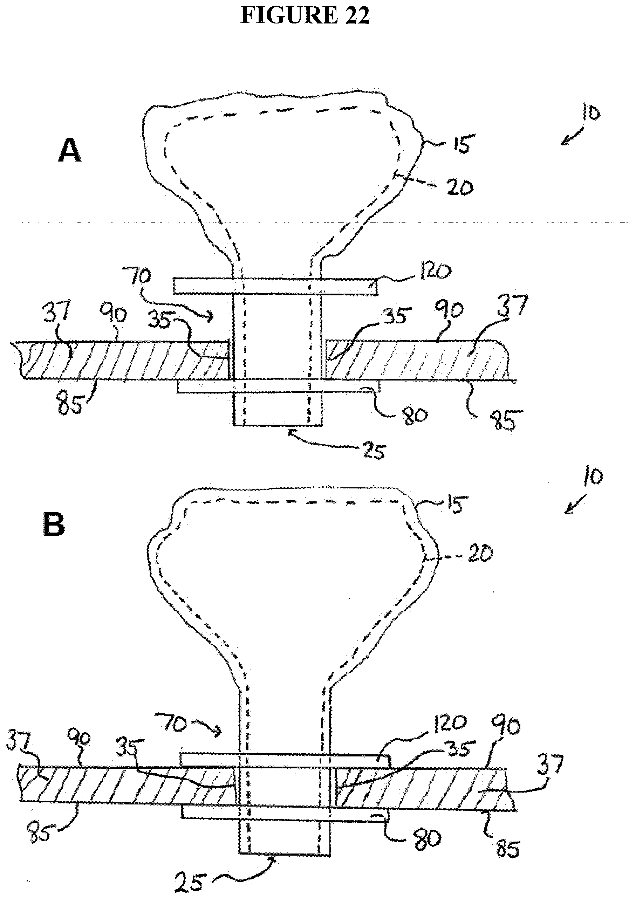

Figure 22A is a side view of the implant insertion device with the second member positioned in a non-engagement position.
Figure 22B is a side view of the implant insertion device of Figure 22A with the second member positioned in an engagement position.

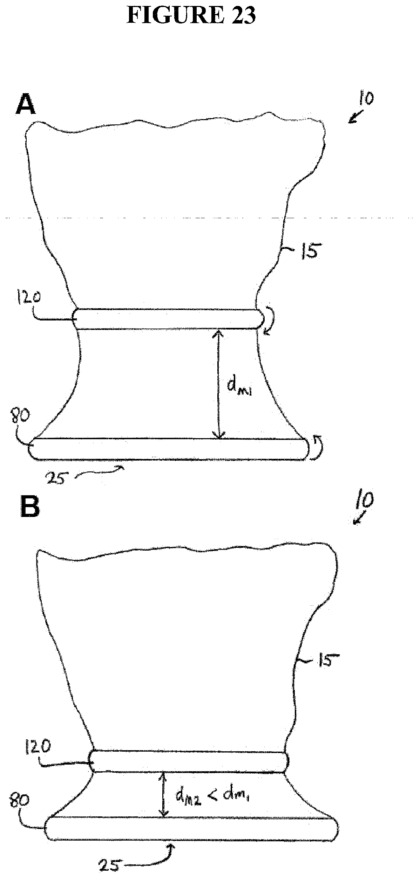

Figure 23A is a side view of the implant insertion device with the first and second members positioned in an extended position.
Figure 23B is a side view of the implant insertion device of Figure 23A with the first and second members positioned in a retracted position.

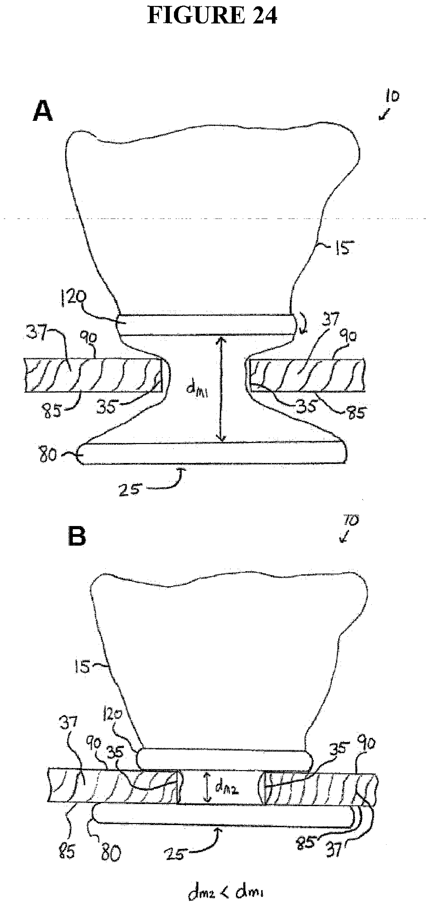

Figure 24A is a side view of the implant insertion device with the first and second members positioned in an extended (non-engagement) position.
Figure 24B is a side view of the implant insertion device of Figure 24A with the first and second members positioned in a retracted (engagement) position.

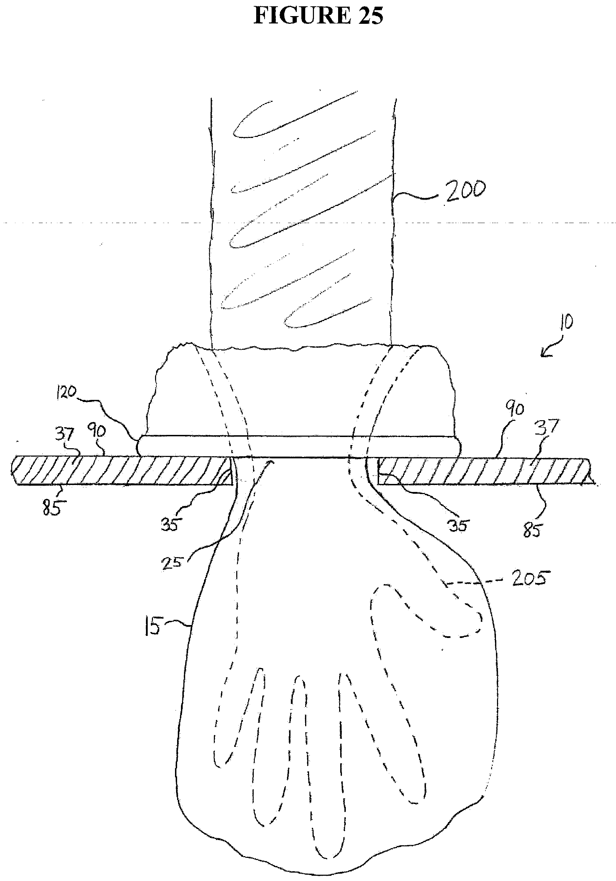

Figure 25 is a side view of the implant insertion device with an invertible body.

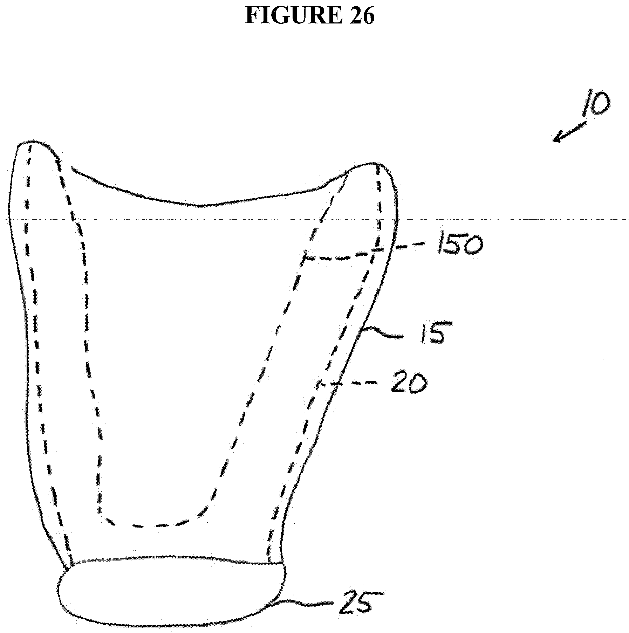

Figure 26 is a side view of the implant insertion device with an access port.

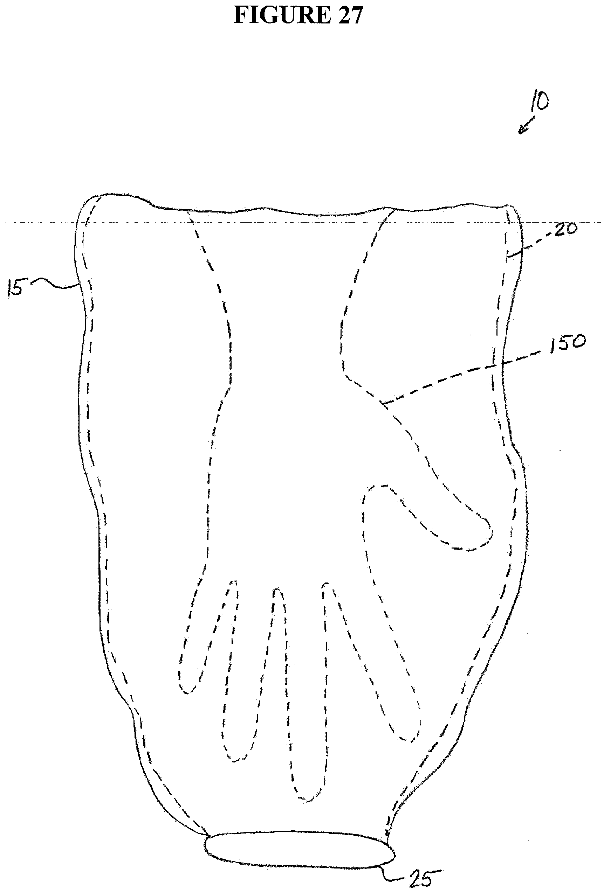

Figure 27 is a side view of the implant insertion device with a glove shaped access port.

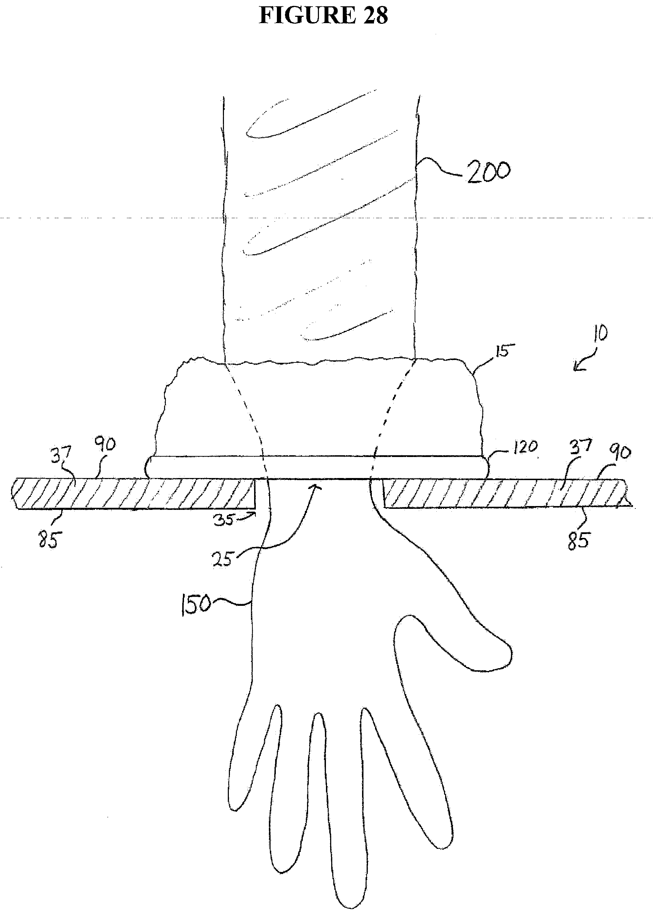

Figure 28 is a side view of the implant insertion device comprising an access port insertable through the incision.

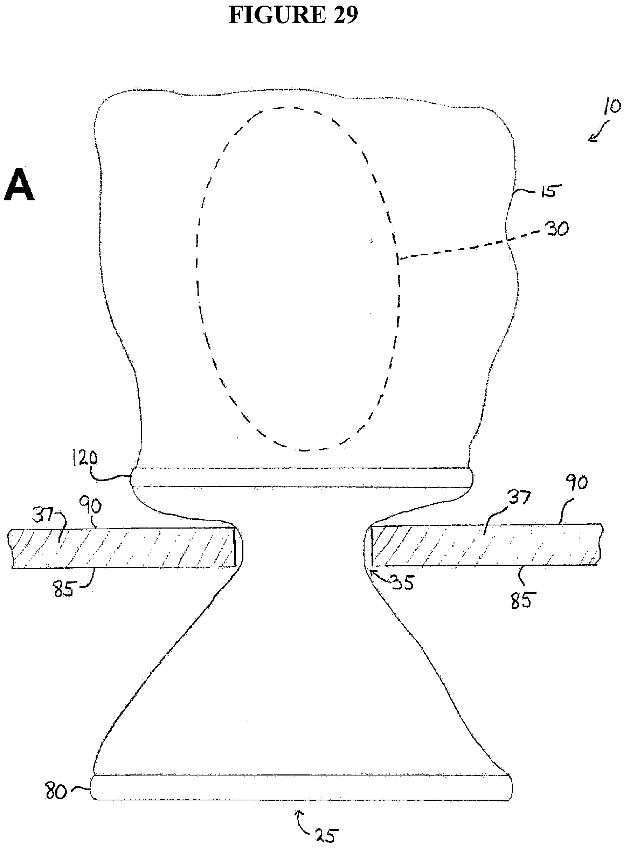

Figure 29A is a side view of the implant insertion device with an implant positioned therein.

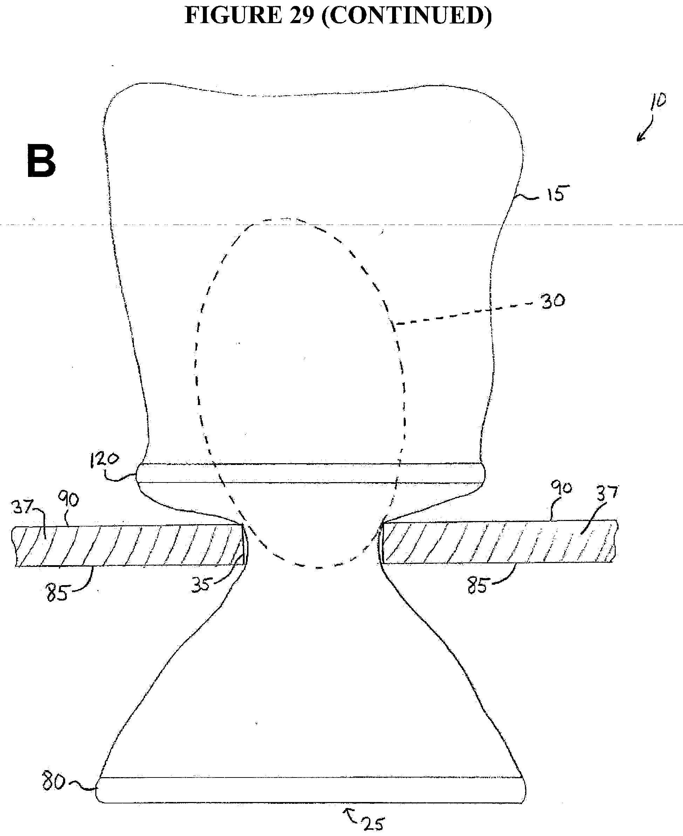

Figure 29B is a side view of the implant insertion device of Figure 29A with the implant being inserted in the incision.

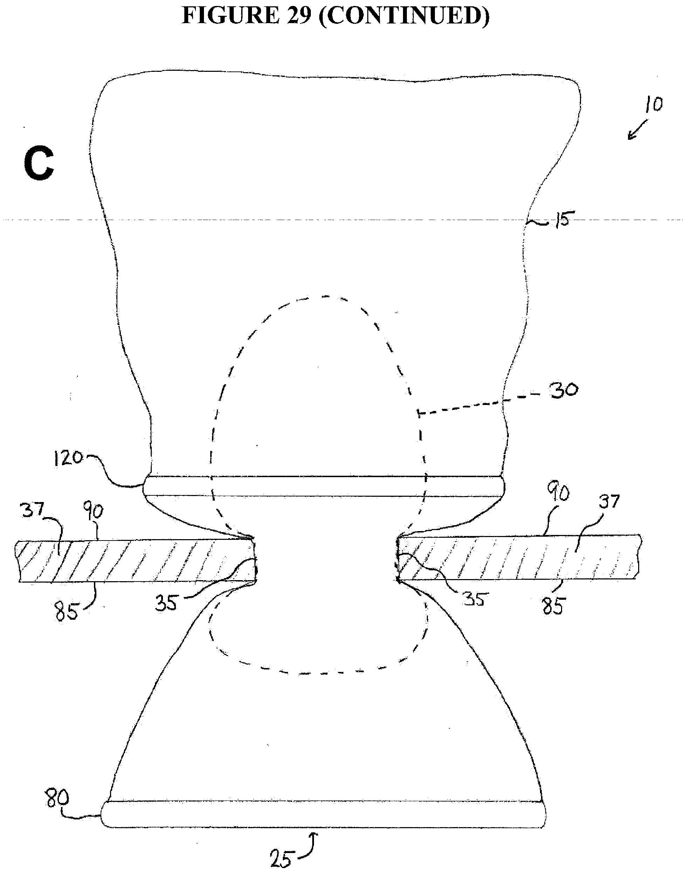

Figure 29C is a side view of the device of Figure 29A with the implant partially inserted through the incision.

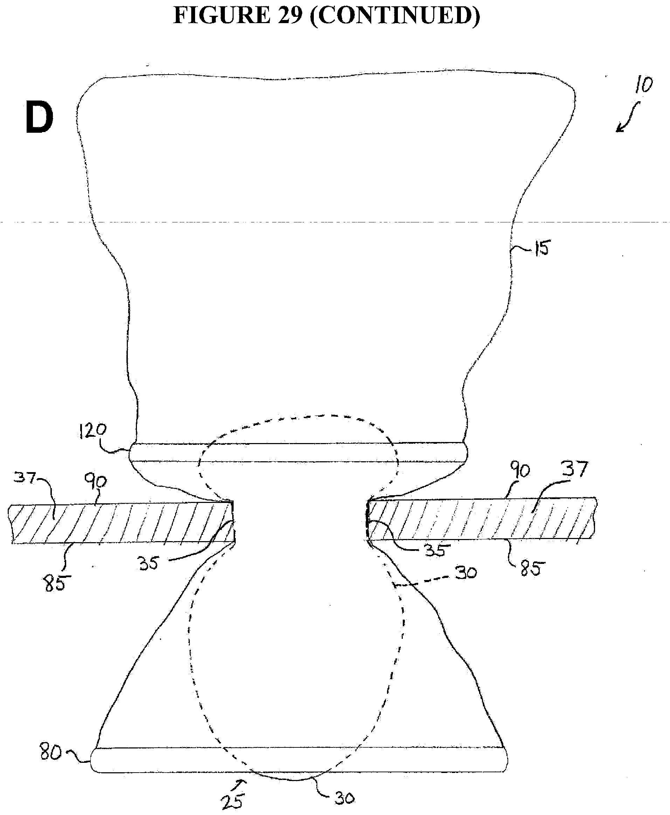

Figure 29D is a side view of the device of Figure 29A with the implant partially inserted through the incision.

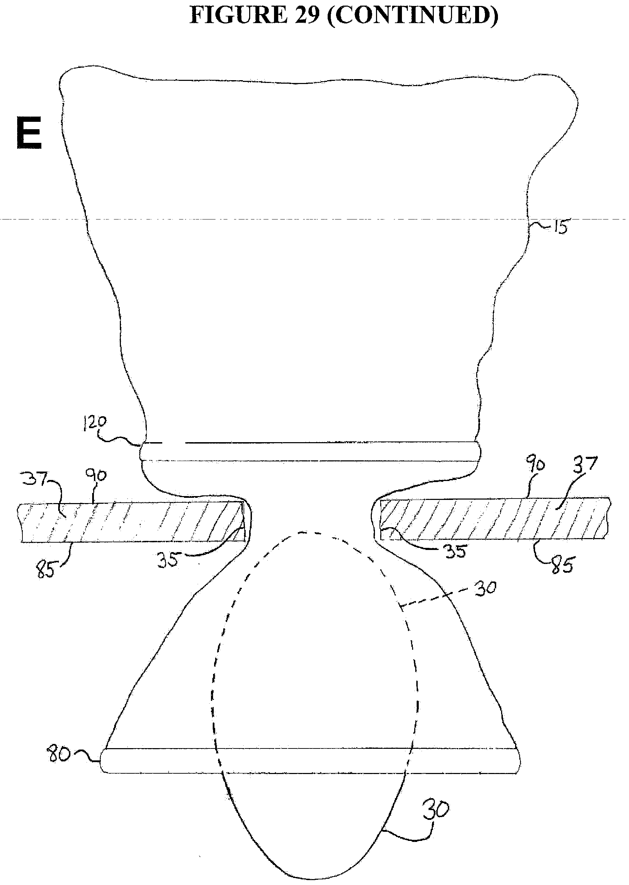

Figure 29E is a side view of the device of Figure 29A with the implant inserted through the incision.

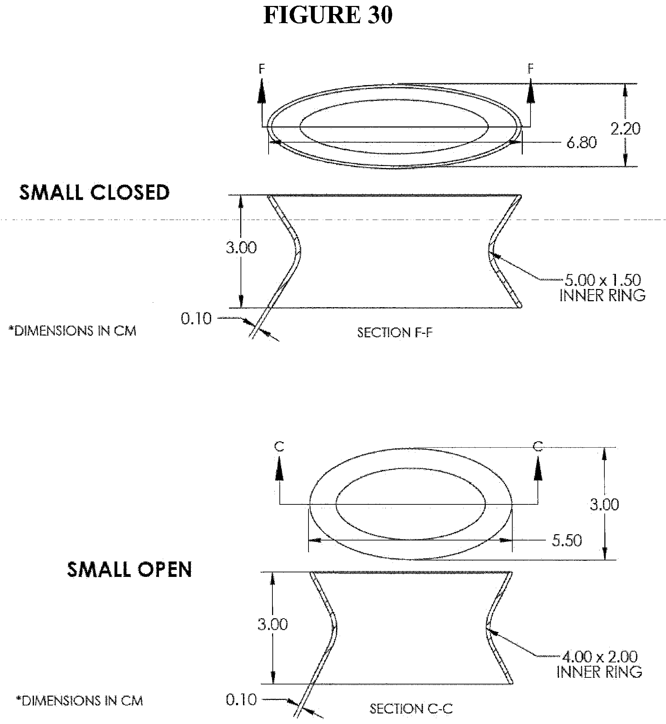

Figure 30 shows a smaller sized embodiment of the current invention both in open and closed positions.

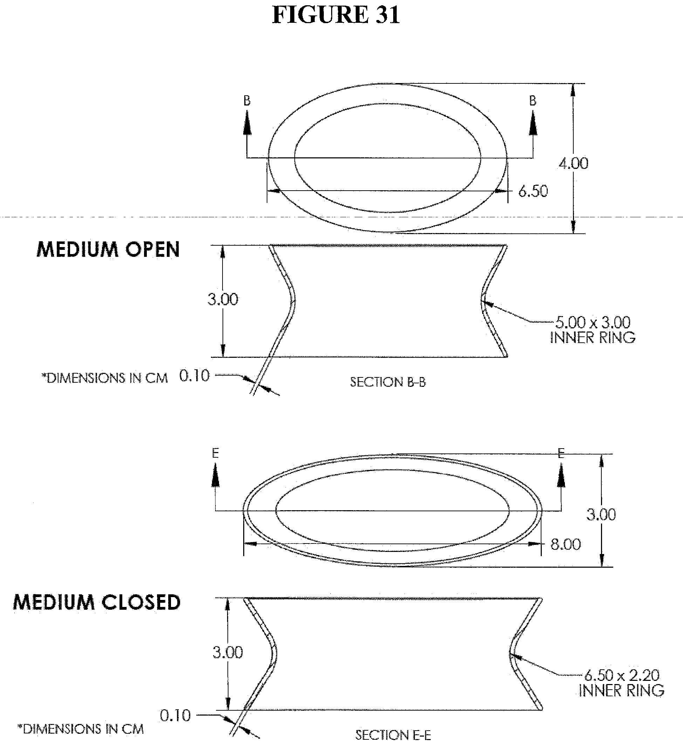

Figure 31 shows a medium sized embodiment of the current invention both in open and closed positions.

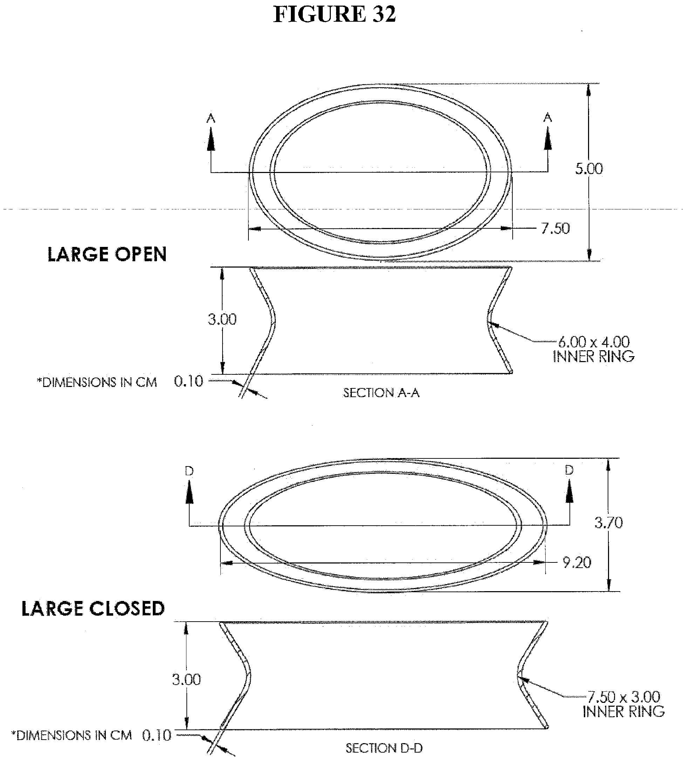

Figure 32 shows a large sized embodiment of the current invention both in open and closed positions.

### DETAILED DESCRIPTION OF THE PREFERRED EMBODIMENTS

While the present invention is described with reference to embodiments described herein, it should be clear that the present invention is not limited to such embodiments. Therefore, the description of the embodiments herein is merely illustrative of the present invention and will not limit the scope of the invention as claimed.

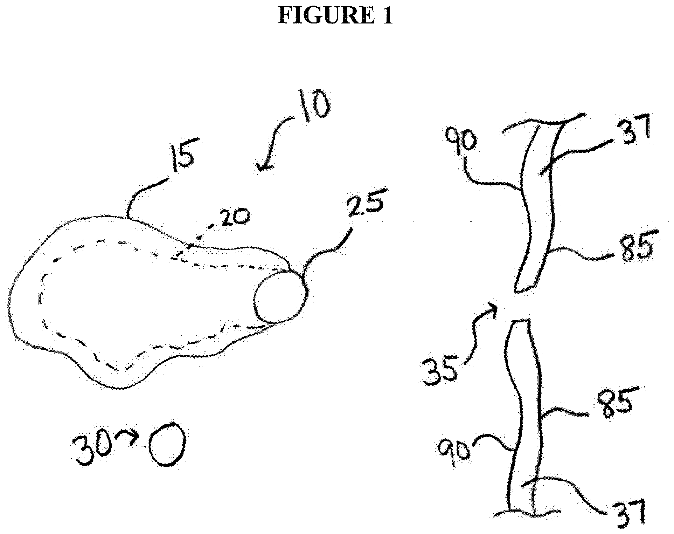

As shown in Figure 1, an implant insertion device 10 (hereinafter referred to as the "device 10") is provided comprising a body 15 defining a cavity 20 therein that is accessible via an aperture 25. An implant 30 is positionable in the cavity 20 and the aperture 25 is substantially coaxially alignable with an incision 35 in the tissue 37 of a human or animal (hereinafter referred to as the "patient") for insertion of the implant 30 therein.

The device 10 may be used for the insertion of a breast implant into a surgical pocket formed in the patient. The breast implant may be any type, including, but not limited to, saline and silicone breast implants. In a non-limiting example, saline breast implants are generally inserted through the incision 35 ranging from about two centimeters to about three centimeters. For silicone breast implants, the incision 35 ranges from about five centimeters and above. It is to be understood, however, that the device 10 may be used with incisions 35 having larger and smaller sizes. In addition, although the implant 30 is described herein as a breast implant, it is to be understood that the device 10 is not limited to breast implants, and may be used to insert any type of implant 30 in the patient.

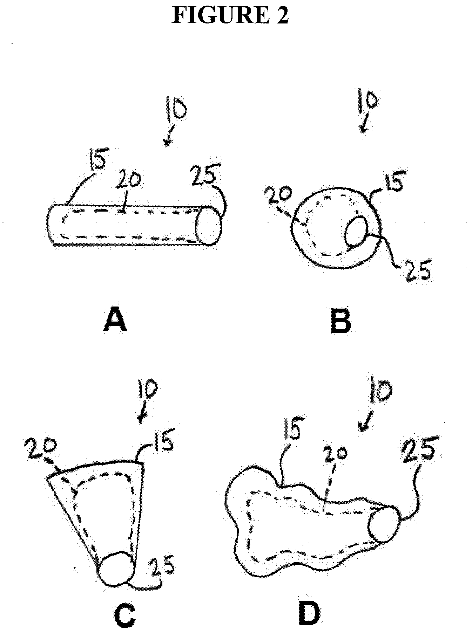

The body 15 may be provided in a variety of shapes and materials. In the non-limiting examples as shown in Figure 2A-D, the body 15 may be substantially cylindrical, spherical, funnel shaped, or bag-like. The body 15 may be comprised of metal, polymers or plastics, composites, and combinations thereof, and may be covered with a friction reducing coating to minimize trauma to the implant 30 and the tissue 37. The body 15 may be coated with a lubricant including, but not limited to, silicone. In a non-limiting example, the body 15 may be of metal, plastic, polymer, fabrics, composites, and combinations thereof. In a non-limiting example, the body 15 may include, but is not limited to, Mylar®, plastics made from Tygon® brands of plastics, vinyls, polyvinyl chloride, ethylene and alpha-olefin copolymers, silicone, and the like. It is to be understood that the body 15 may be impregnated with an antimicrobial.

The cavity 20 may extend a portion of or the entire length of the body 15. In an embodiment, at least a portion of the body 15 is flexible and capable of allowing a user 200, such as a physician, to manipulate or otherwise apply pressure to the implant 30 via the body 15 when positioned in the cavity 20 by hand or with an instrument to transfer the implant 30 from the cavity 20 and into the patient via the incision 35. Accordingly, the implant 30 may be inserted in the patient without exposing the implant 30 or the patient and the surgical pocket to contamination from a variety of sources, including but not limited to, the physician's gloves, hands, retractors, and the patient's skin, thereby reducing the chance of infection and bacterial seeding of the implant 30.

It is to be understood that the implant 30 is insertable in the cavity 20 via the aperture 25. The aperture 25 may be smaller (or have a smaller diameter) than the implant 30, substantially same as the implant 30, or larger than the implant 30. In a non-limiting example as best shown in Figure 15A and Figure 15B, the aperture 25 may be smaller than the implant 30 and the body 15 may be resistant to stretching, thereby causing the implant 30 to extrude through the aperture 25. In a non-limiting example as best shown in Figure 16A and Figure 16B, a portion of the body 15 adjacent to or surrounding the aperture 25 may be stretchable or expandable to increase the size or diameter of the aperture 25 for insertion or removal of the implant 30 therethrough without substantially compressing or extruding the implant 30, thereby reducing trauma to the implant 30.

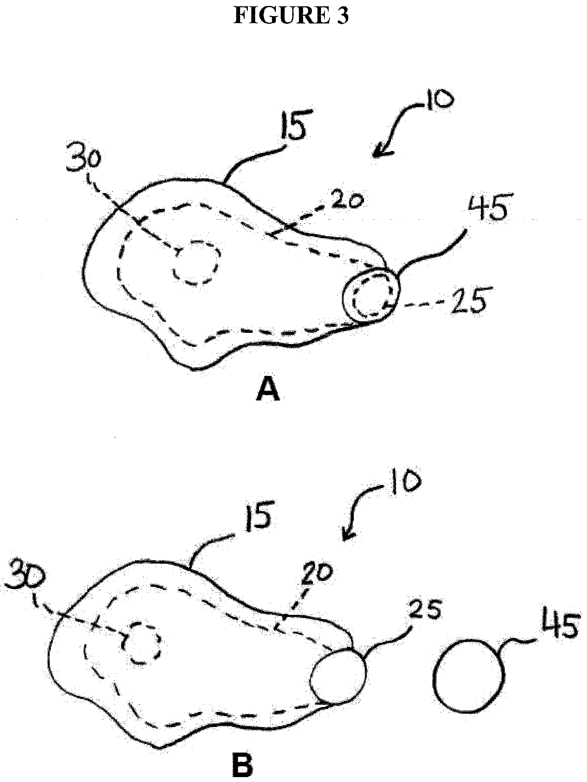

As best shown in Figure 3A, the device 10 may be provided with an implant 30 positioned in the cavity 20. A cover 45 may be provided for the aperture 25, for example, to maintain the sterility of at least a portion of the body 15, the cavity 20, the implant 30, and combinations thereof. As shown in Figure 3B, the cover 45 may be removed before inserting the implant 30 through the incision 35.

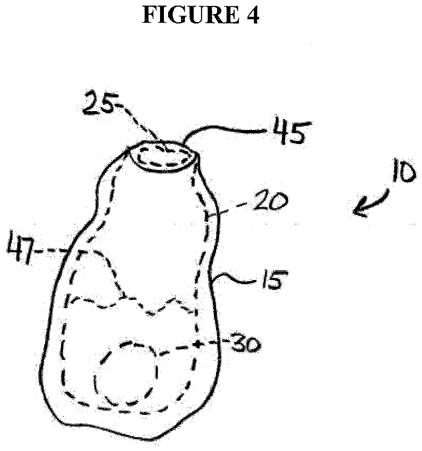

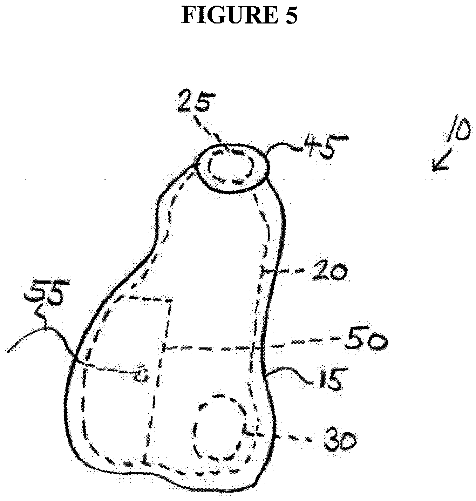

As shown in Figure 4, a fluid 47 may be provided in the cavity 20, for example, to sterilize the implant 30. The fluid 47 may include, but is not limited to, lubricant, disinfectant, sterilizer, antibiotic, antimicrobial, and combinations thereof. The disinfectant 47 may be provided in contact with the implant 30 as shown in Figure 4, or in a compartment 50 capable of being opened to place the compartment 50 in fluid communication with the cavity 20 as shown in Figure 5. The compartment 50 may be opened prior to insertion of the implant 30 through the incision 35 to release the fluid 47 into the cavity 20. In a non-limiting example, the compartment 50 may be opened by applying pressure to the body 15, for example, by pinching between the user's 200 fingers.

A switch 55 may be provided outside of the cavity 20 as shown in Figure 5 that may be activated to open the compartment 50. In a non-limiting example, the switch 55 may be a string that can be pulled to open the compartment 50. It is to be understood, however, that a variety of configurations may be used to open the compartment 50.

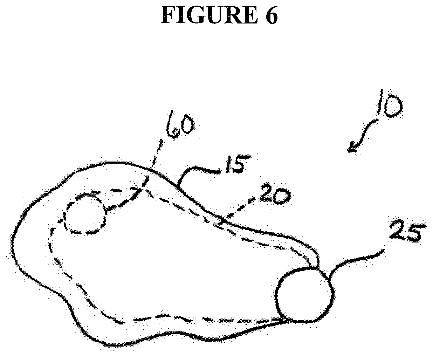

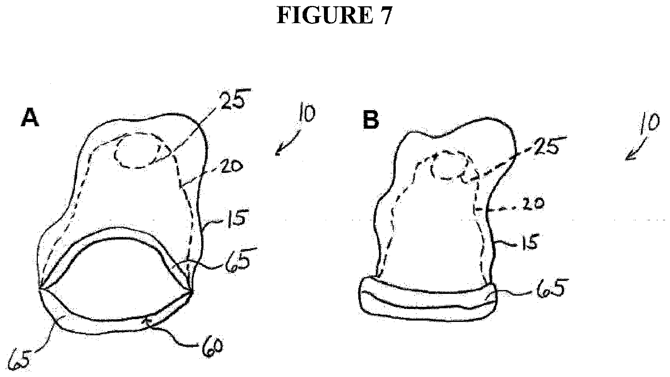

As shown in Figure 6, the body 15 may be provided with a second aperture 60 capable of receiving the implant 30 for placement in the cavity 20. In a non-limiting example as best shown in Figure 7A and Figure 7B, the second aperture 60 may be provided with a closure 65 that may be selectively opened (Figure 7A) and closed (Figure 7B) for access to the cavity 20. The closure 65 may include, but is not limited to, a Zip-loc closure, a suture, zipper, button, adhesive, strings for tying for tying the second aperture 60 closed, and combinations thereof. The second aperture 60 may have a diameter less than, substantially equal to, or greater than the diameter of the implant 30.

In an embodiment as shown in Figure 8, the device 10 may be provided with a neck 70 extending from the body 15 and capable of engaging the incision 35 to facilitate insertion of the implant 30 through the incision 35. As shown in Figure 9A, Figure 9B, and Figure 9C the neck 70 may comprise two or more legs 75 that are positionable in a first non-engagement position for insertion in the incision 35 and a second engagement position to maintain the incision 35 in an open position (having a size or diameter d₁). As shown in Figure 9B and Figure 9C, the neck 70 may stretch or expand the size or diameter d₁ of the incision 35 while in the engagement position to a larger diameter d₂. As shown in Figure 9A, the legs 75 (or neck 70) may be inserted in or through the incision 35 without contacting an external surface 90 of the skin (or substantially) any portion of the incision 35 walls), thereby minimizing the introduction of bacteria or other foreign matter to the implant 30 or in the patient's body (including the surgical pocket).

Although shown as being substantially rod shaped, it is to be understood that the legs 75 (or the neck 70) may be any shape capable of insertion in or through the incision 35. Although shown as extending substantially perpendicularly outward from the body 15, the legs 75 (or neck 70) may extend outward from the body 15 at any angle. Although shown as extending outward from the body 15 substantially parallel to each other, the legs 75 may extend outward from the body 15 at any angle with respect to each other.

The legs 75 (or neck 70) may be biased such that the legs 75 may be compressed to the first non-engagement position for insertion in the incision 35. When released, the legs 75 extend outward from each other to engage the tissue 37 surrounding the incision 35 to maintain the incision 35 in an open position or increase the size of the incision 35. In a non-limiting example, the legs 75 may comprise a shape memory material to provide the biasing force to the legs 75. In a non-limiting example as shown in Figure 14, a biasing member 105 may be provided to provide the biasing force to the legs 75. In a non-limiting example, the biasing member 105 may be a spring. Although the biasing member 105 is shown as being positioned between the legs 75, it is to be understood that the biasing member 105 may be positioned anywhere on the device 10 to provide the biasing force. In addition, it is to be understood that a variety of materials and configurations may be used to provide the biasing force to the legs 75.

A locking mechanism (not shown) may be provided to lock the legs 75 in the first non-engagement position, the second engagement position, or any position therebetween. In a non-limiting example, the locking mechanism may be used to prevent the legs 75 from being compressed inwardly from the second engagement position to prevent accidental withdrawal from the incision 35.

As shown in Figure 10A, Figure 10B, and Figure 10C, the neck 70 may be provided with one or more engagement members 80 (hereinafter referred to as "the first member 80"). The first member 80 is capable of engaging the patient to prevent removal of the device 10 from the patient during insertion of the implant 30, to maintain alignment of the aperture 25 with the incision 35, to prevent over insertion of the device 10 in the patient, and combinations thereof. The first member 80 may extend outward from the body 15 or the neck 70 to engage an internal part of the patient's body. In a non-limiting example, the first member 80 may engage an internal surface 85 opposite the external surface 90 or a portion of a surgical pocket (not shown) in the patient formed for the placement of the implant 30 therein.

As shown in Figure 10A, Figure 10B and Figure 10C, the first member 80 may extend substantially perpendicularly outward from the legs 75 (or neck 70). It is to be understood, however, that the first member 80 may extend at any angle outward from the legs 75 (or neck 70) and may be curved or otherwise shaped to conform to the shape of the tissue to which it will engage.

As shown in Figure 11A and Figure 11B, the first member 80 may be selectively moveable between a non-engagement position (Figure 11A) and an engagement position (Figure 11B). The first member 80 may be moved by using one or more actuators 95, such as a button, tab, or the like. The first member 80 may be positioned in the non-engagement position (Figure 11A) for insertion through the incision 35 and extended to the engagement position (Figure 11B) to engage the internal surface 85 for insertion of the implant 30 through the incision 35.

In a non-limiting example, the first member 80 may be secured to the body 15. The first member 80 may be comprised of metal, polymer, plastic, fabrics, composites, and combinations thereof. It is to be understood that the first member 80 may be rigid, compressible, foldable, expandable, or stretchable. As shown in Figure 12, the first member 80 may be substantially ring shaped and may extend outward from a flexible, bag-like body 15. In a non-limiting example, the first member 80 may be comprised of a flexible material, including but not limited to a polymer, capable of being compressed or folded for insertion through the incision 35.

As best shown in Figure 13A, one or more arms 100 may be provided extending from the first member 80. As best shown in Figure 13B, the arms 100 may be manipulated, for example by squeezing together, to compress or fold the first member 80, the neck 70, the portion of the body 15 surrounding the aperture 25, or any combination thereof, to the non-engagement position for insertion through the incision 35. As shown in Figure 13C and Figure 13D, the first member 80, the neck 70, the portion the body 15 surrounding the aperture 25, or any combination thereof, may be folded to the non-engagement position for insertion through the incision 35. Although not shown in Figure 13C and Figure 13D, it is to be understood that one or more arms 100 may be provided.

Accordingly, the first member 80, the neck 70, the portion the body 15 surrounding the aperture 25, or any combination thereof may be inserted in or through the incision 35 to reduce exposure of the patient and the implant 30 to contamination from the physician's gloves, hands, retractors and the like, thereby reducing the risk of infection or bacteria seeding. The arms 100 (if provided), the neck 70, the first member 80, the body 15, or any combination thereof, may be released to allow the first member 80, the neck 70, the portion of the body 15 surrounding the aperture 25, or any combination thereof, to return to the engagement position as shown in Figure 13A.

In an embodiment, an engagement member 120 (hereinafter referred to as "the second member 120") may be provided for engaging the external surface 90 of the patient's skin. As shown in Figure 17A and Figure 17B, the first member 120 may be secured to the body 15 or the neck 70. As shown in Figure 18A and Figure 18B, the second member 120 may engage the external surface 90 to secure the device 10 to the patient, to maintain alignment of the aperture 25 with the incision 35, to prevent over insertion of the any portion of the device 10 in the incision 35 (for example, during insertion of the implant 30), and combinations thereof. Over insertion of the device 10 in the patient may introduce bacteria or foreign matter in the patient, or cause trauma to the patient.

In a non-limiting example, the second member 120 may be capable of providing a vacuum when engaged with the external surface 90 to secure the device 10 thereto. It is to be understood, however, that other configurations of the second member 120 may be used to secure the device 10 to the external surface 90, including, but not limited to clamps, ribbons, and the like. Although shown as substantially ring shaped, the second member 120 may be any shape capable of engaging the external surface 90. The second member 120 may be comprised of metal, polymer, plastic, fabrics, composites, and combinations thereof. It is to be understood that the second member 120 may be rigid, compressible, expandable or stretchable.

It is to be understood that the second member 120 may be integral with the body 15 or the neck 70 and may be removeably secured to the body 15 or the neck 70. As best shown in Figure 19A and Figure 19B, the second member 120 may be provided in combination with the first member 80. As shown in Figure 20A and Figure 20B, the second member 120 may engage the external surface 90 and the first member 80 may engage the internal surface 85. In a non-limiting example, the second member 120 and the first member 80 may be selectively positionable or biased toward each other to sandwich or compress the tissue 37 therebetween.

The second member 120, the first member 80, or both the second member 120 and the first member 80 may be selectively positionable along the neck 70 or body 15. In a non-limiting example as shown in Figure 21A, Figure 21B, and Figure 21C, the neck 70 may be provided with a series of apertures 130. The second member 120, the first member 80, or both the second member 120 and the first member 80 may be provided with an actuator 140, such as a pin, to selectively engage the apertures 130 to lock the second member 120 or the first member 80 at a desired position on the neck 70.

In another illustrative example, as shown in Figure 23A and Figure 23B, either or both of the members 120 and 80 may be rotated to wrap the body 15 (and/or neck 70) thereabout to decrease the distance dₘ₁ and dₘ₂ therebetween (where dₘ₁ > dₘ₂). As shown in FIGS. Figure 24A and Figure 24B, the second member 120 may be rotated after insertion of the first member 80 through the incision 35 to selectively engage the tissue 37 therebetween. It is to be understood, however, that the foregoing illustrative examples are not limiting and that a variety of configurations may be provided for selectively positioning the members 120 and 80 along the body 15 or neck 70.

As shown in Figure 22A and Figure 22B, the first member 80 may be inserted through the incision 35 to engage the internal surface 85. As shown in Figure 22B, the second member 120 may be selectively positioned to engage the external surface 90 to sandwich or compress the tissue 37 therebetween to, for example, secure the device 10 to the tissue 37, to maintain alignment of the aperture 25 with the incision 35, prevent over insertion of the device 10 through the incision 35, and combinations thereof.

As shown in Figure 25, the body 15 may be capable of being inverted to allow the user 200 to insert their hand 205 (or a portion thereof) through the incision 35 to manipulate or otherwise position the implant 30 in the surgical pocket without directly contacting the skin, the implant 30, or the surgical pocket. Accordingly, the user 200 may minimize the risk of introducing foreign matter (including but not exclusive to lint from surgical towels or powder from surgical gloves) or bacteria on the implant 30 and in the patient and the surgical pocket.

In an embodiment as shown in Figure 26, a port 150 is provided for insertion of a hand or tool in the cavity 20. As best shown in Figure 27, the port 150 may be shaped like a glove to facilitate insertion of the user's hand 205 therein. The port 150 provides access to the cavity 20 to allow the user 200 to manipulate the implant 30 therein, and allows the user 200 to transfer the implant 30 through the aperture 25 and the incision 35. As best shown in Figure 28, the port 150 may allow the user 200 to insert at least a portion of their hand 205 through the incision 35, for example, to manipulate the implant 30 in the surgical pocket to facilitate proper positioning. It is to be understood that the port 150 may be comprised of the same material as the body 15. In a non-limiting example, the body 15, the neck 70, and combinations thereof may be comprised of a rigid material and the port 150 may be comprised of a flexible material.

Turning to the device 10, an illustrative example of how to use the device 10 as illustrated in Figure 1-Figure 29D is set forth below. As best shown in Figure 8A, Figure 9A, Figure 10A, Figure 13B, Figure 13C, and Figure 13D the neck 70 (or a portion of the body 15) may be provided in a non-engagement position for insertion in the incision 35 without contacting (or substantially contacting) the external surface 90 to, for example, minimize the introduction of foreign material in the patient and the surgical pocket. As best shown in Figure 8B, Figure 8C, Figure 9B, Figure 9C, Figure 10B, and Figure 10C, the neck 70 (or a portion of the body 15) may be moveable from the non-engagement position to the engagement position to selectively engage the incision 35 walls to maintain the incision 35 in an open position with the aperture 25 substantially coaxially aligned with the incision 35.

As shown in Figure 22A, the first member 80 may be provided to engage the internal surface 85 to, for example, prevent withdrawal of the device 10 from the incision 35 during insertion of the implant. The second member 120 may be provided to engage the external surface 90 to, for example, secure the device 10 to the external surface 90 to maintain alignment of the aperture 25 with the incision 35, to prevent over insertion of the device 10 in the patient, and combinations thereof. As shown in Figure 22A and Figure 22B, the second member 120 may be selectively positionable from a first non-engagement position (Figure 22A) to a second engagement position (Figure 22B).

As best shown in Figure 29A and Figure 29B, the implant 30 may be aligned with the incision 35 for insertion therethrough. It is to be understood that the body 15 (or a portion thereof) may be flexible and allow the user 200 to manipulate or transfer the implant 30 through the incision 35, as shown in Figure 29C, Figure 29D, and Figure 29E, without directly contacting the implant 30, to minimize the introduction of foreign matter and bacteria to the implant 30 and in the patient and the surgical pocket. As best shown in Figure 29B, Figure 29C, Figure 29D, and Figure 29E, the aperture 25 may be larger than the implant 30 and the body 15 may be flexible and shaped so as not to constrict or otherwise compress the implant 30 during insertion through the incision 35. Accordingly, the device 10 may limit compression or trauma exerted on the implant 30 to that imposed thereon by the tissue 37 surrounding the incision 35, the fingers, or tools of the user 200 used to transfer the implant 30 therethrough.

## Claims

1. An implant insertion device (10), comprising:
a) a foldable or stretchable body (15) comprising an interior cavity (20) capable of receiving an implant therein and an aperture (25) in communication with the cavity (20),
b) a neck (70) having an end extending from the body (15),
c) a first member (80) extending from the body (15), wherein the first member comprises a flexible ring, and
d) a second member (120) extending from the body (15), wherein the second member comprises a flexible ring.

2. The device of claim 1, wherein said first member (80) is configured to engage a tissue (37) of a patient.

3. The device of claim 1 wherein the neck (70) comprises two or more legs (75) selectively positionable from a first non-engagement position to a second engagement position.

4. The device of claim 2 where the first member (80) is configured to engage the patient's external surface (90).

5. The device of claim 2 wherein the first member (80) is configured to engage the patient's internal tissue (85) surface.

6. The device of claim 1 wherein the second member (120) is configured to engage the patient's external skin surface (90).

7. The device of claim 1 wherein the second member (120) is selectively positionable from a first non-engagement position to a second engagement position

8. The device of claim 6 where the second member (120) is configured to provide a vacuum when engaged with the external surface (90) to secure the device (10) thereto.

9. The device of claim 1 wherein the second member (120) has a diameter selected from the group consisting of greater than the diameter of the first member (80), substantially equal to the diameter of the first member (80), and smaller than the diameter of the first member (80).

## Patentansprüche

1. Vorrichtung (10) zum Einsetzen von Implantaten, umfassend:
a) einen faltbaren oder ausdehnbaren Körper (15), umfassend einen inneren Hohlraum (20), welcher ein Implantat darin aufnehmen kann und eine Öffnung (25), welche mit dem Hohlraum (20) in Verbindung steht,
b) einen Hals (70), welcher ein sich vom Körper (15) erstreckendes Ende aufweist,
c) ein erstes Element (80), welches sich vom Körper (15) erstreckt, wobei das erste Element einen flexiblen Ring umfasst, und
d) ein zweites Element (120), welches sich vom Körper (15) erstreckt, wobei das zweite Element einen flexiblen Ring umfasst.

2. Vorrichtung nach Anspruch 1, wobei das erste Element (80) konfiguriert ist, um in das Gewebe (37) eines Patienten einzugreifen.

3. Vorrichtung nach Anspruch 1, wobei der Hals (70) zwei oder mehr Beine (75) umfasst, welche selektiv von einer ersten nicht eingreifenden Position in eine zweite eingreifende Position verstellbar sind.

4. Vorrichtung nach Anspruch 2, wobei das erste Element (80) konfiguriert ist, um in die Außenfläche (90) des Patienten einzugreifen.

5. Vorrichtung nach Anspruch 2, wobei das erste Element (80) konfiguriert ist, um in die innere Fläche des Gewebes (85) des Patienten einzugreifen.

6. Vorrichtung nach Anspruch 1, wobei das zweite Element (120) konfiguriert ist, um in die äußere Hautfläche (90) des Patienten einzugreifen.

7. Vorrichtung nach Anspruch 1, wobei das zweite Element (120) selektiv von einer ersten nicht eingreifenden Position in eine zweite eingreifende Position verstellbar ist.

8. Vorrichtung nach Anspruch 6, wobei das zweite Element (120) konfiguriert ist, um ein Vakuum zu erzeugen, wenn es in die äußere Fläche (90) eingreift, um die Vorrichtung (10) an dieselbe zu befestigen.

9. Vorrichtung nach Anspruch 1, wobei das zweite Element (120) einen Durchmesser aufweist, welcher so ausgewählt ist, dass er größer als der Durchmesser des ersten Elements (80), im Wesentlichen gleich dem Durchmesser des ersten Elements (80) oder kleiner als der Durchmesser des ersten Elements (80) ist.

## Revendications

1. Dispositif d'insertion d'implant (10), comprenant :
a) un corps pliable ou étirable (15) comprenant une cavité intérieure (20) capable de recevoir un implant et une ouverture (25) en communication avec la cavité (20),
b) un col (70) ayant une extrémité s'étendant à partir du corps (15),
c) un premier élément (80) s'étendant à partir du corps (15), dans lequel le premier élément comprend une bague souple, et
d) un deuxième élément (120) s'étendant à partir du corps (15), dans lequel le deuxième élément comprend une bague flexible.

2. Dispositif selon la revendication 1, dans lequel ledit premier élément (80) est configuré pour se mettre en prise avec un tissu (37) d'un patient.

3. Dispositif selon la revendication 1, dans lequel le col (70) comprend deux pattes (75) ou plus positionnables sélectivement d'une position de non-mise en prise à une deuxième position de mise en prise.

4. Dispositif selon la revendication 2, dans lequel le premier élément (80) est configuré pour venir en prise avec la surface externe (90) du patient.

5. Dispositif selon la revendication 2, dans lequel le premier élément (80) est configuré pour se mettre en prise avec la surface du tissu interne (85) du patient.

6. Dispositif selon la revendication 1, dans lequel le deuxième élément (120) est configuré pour se mettre en prise avec la surface cutanée externe (90) du patient.

7. Dispositif selon la revendication 1, dans lequel le deuxième élément (120) est positionnable sélectivement d'une première position de non-mise en prise à une deuxième position de mise en prise.

8. Dispositif selon la revendication 6, dans lequel le deuxième élément (120) est configuré pour fournir un vide lorsqu'il est mis en prise avec la surface externe (90) pour fixer le dispositif (10) à celle-ci.

9. Dispositif selon la revendication 1, dans lequel le deuxième élément (120) a un diamètre sélectionné dans le groupe constitué par « plus grand que le diamètre du premier élément (80) », « sensiblement égal au diamètre du premier élément (80) » et « plus petit que le diamètre du premier élément (80) ».
